(19) Europäisches Patentamt
European Patent Office
Office européen des brevets

(11) Publication number: 0 034 281
A2

(12) # EUROPEAN PATENT APPLICATION

(21) Application number: 81100632.9

(22) Date of filing: 29.01.81

(51) Int. Cl.[3]: A 01 N 51/00
C 07 C 111/00, C 07 D 231/04
C 07 D 231/06, C 07 D 231/12
C 07 C 149/30, C 07 C 149/243
C 07 D 307/14, C 07 D 231/18
C 07 D 231/38, C 07 D 295/02

(30) Priority: 19.02.80 US 122643
18.08.80 US 179335

(43) Date of publication of application:
26.08.81 Bulletin 81/34

(84) Designated Contracting States:
BE CH DE FR GB IT LI NL SE

(71) Applicant: American Cyanamid Company
1937 West Main Street
Stamford Connecticut 06904(US)

(72) Inventor: O'Neal, Thomas Denny
365 Cold Soil Road
Princeton New Jersey 08540(US)

(72) Inventor: Bhalla, Prithvi Raj
170 Hickory Corner Road
Hightstown New Jersey 08520(US)

(72) Inventor: Cross, Barrington
27 Toth Lane
Rocky Hill New Jersey 08553(US)

(74) Representative: Wächtershäuser, Günter, Dr.
Gewürzmühlstrasse 5
D-8000 München 22(DE)

(54) **Method for the control of stem growth and stem stiffness of selected crops.**

(57) Novel inorganic and organic salts of symmetrical trisubstituted-N-nitroaniline compounds which are effective for increasing axillary branching, improving canopy and enhancing flowering of herbaceous ornamental plants and graminaceous crops, leguminous crops, cotton and sunflowers and effective as yield enhancing agents and lodging inhibitors for the above-said crops.



Croydon Printing Company Ltd.

INORGANIC AND ORGANIC SALTS OF SYMMETRICAL TRISUBSTITUTED-N-NITROANILINE COMPOUNDS USEFUL FOR ENHANCING AXILLARY BRANCHING, CANOPY, FLOWERING AND CROP YIELD OF PLANTS AND AS LODGING INHIBITORS THEREFOR

The present invention relates to plant regulating salts of 2,4,6-trisubstituted-N-nitroanilines represented by formula (I):

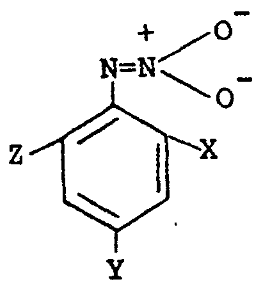 · $M^+$ (I)

wherein X,Y and Z each are halogen , $C_1$-$C_3$ alkyl, $C_1$-$C_3$ alkoxy, $-CO_2$ alkyl($C_1$-$C_3$), CN, $CF_3$, $-SO_2F$ or $-SO_2CHF_2$.

The cation $M^+$ of formula (I) may be inorganic or organic. When M is inorganic, it is alkali metals, alkaline earth metals, Mn, Fe, Co, Ni, Cu, Zn, Al, Pb or Ag.

When the cation $M^+$ is organic, M is represented by formula (1a)

$$N\ R_1R_2R_3R_4 \qquad (1a)$$

wherein $R_1$ is hydrogen, $C_{13}$-$C_{18}$ alkyl straight chain or branched or $C_3$-$C_6$ alkenyl, $C_3$-$C_6$ alkynyl, $C_3$-$C_6$ cycloalkyl, $R_2$ is hydrogen, $C_{13}$-$C_{18}$ alkyl straight chain or branched or $R_3$ is hydrogen, $C_{13}$-$C_{18}$ alkyl straight chain or branched $R_4$ is hydrogen, $C_{13}$-$C_{18}$ alkyl straight chain or branched or

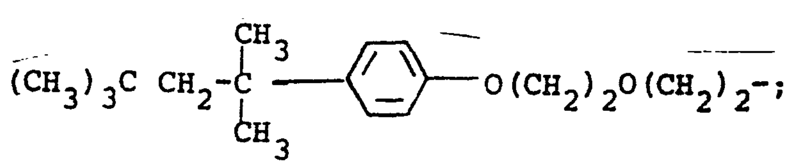

The organic cation $M^+$ may also be represented by formula (1b)

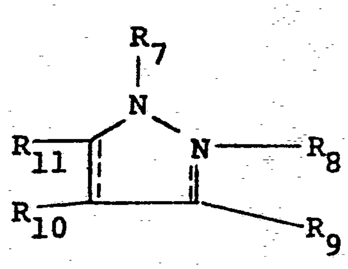

(Ib)

wherein $R_7$ and $R_8$ each are $C_1$-$C_3$alkyl; $R_{10}$ is hydrogen, halogen, $C_1$-$C_3$ alkyl or $C_1$-$C_3$ alkoxy; $R_9$ and $R_{11}$ each are hydrogen $C_1$-$C_6$ alkyl straight chain or branched, $C_3$-$C_6$ cycloalkyl, 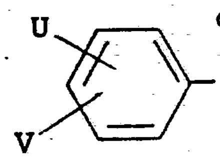 or 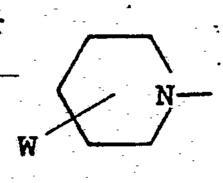; wherein U and V each are hydrogen, halogen, $C_1$-$C_3$ alkyl, $C_1$-$C_3$ alkoxy, CN or $CF_3$; W is hydrogen or $C_1$-$C_3$ alkyl; and each --- symbol represents a single or double bond, wherein if both are single bonds then the cations are pyrazolidinium cations represented by formula (lc)

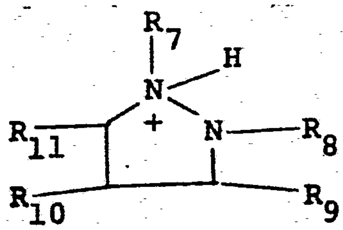

(Ic)

wherein $R_7$ to $R_{11}$ are as defined above; and when one of the --- symbols represents a double bond then the cations are pyrazolinium cations represented by formula (Id)

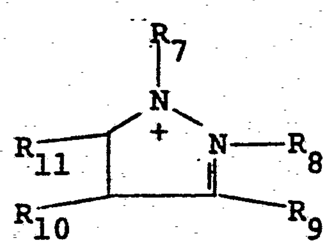

(Id)

wherein $R_7$ to $R_{11}$ are as defined above; and when both --- symbols represent double bonds, then the cations are pyrazolium cations represented by formula (Ie)

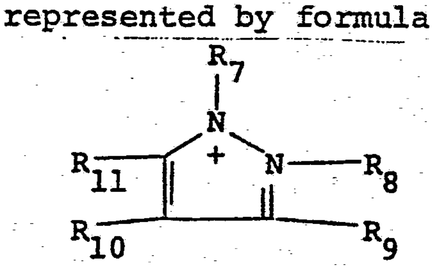

(Ie)

wherein $R_7$ to $R_{11}$ are as defined above.

M may also be represented by Formulae (If) and (Ig)

$$R_{12}-P(R_{13})_3 \qquad (If)$$

wherein $R_{12}$ is $C_1$-$C_6$ alkyl, 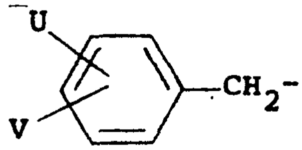 or 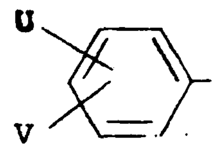

wherein U and V are as herinabove defined; $R_{13}$ is $C_1$-$C_6$ alkyl or 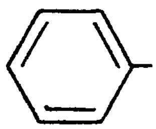 ; and $(C_6H_5)_3S$ (Ig)

The parent invention relates to methods for the control of the relative stem growth of graminaceous crops, leguminous crops and cotton, comprising applying to the foliage stems, roots or seeds of said plants, or to the sóil in which said plants are grown, a plant growth regulating amount of a phenylnitramine or N-nitroaniline having the structure:

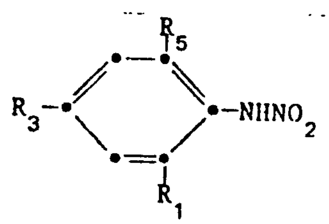

wherein $R_1$ is halogen, $C_1$-$C_4$ alkyl, CN, $CO_2CH_3$, $SO_2CH_3$, $SO_2F$ $SO_2CF_3$, $C_1$-$C_3$ haloalkyl, $C_1$-$C_3$ haloalkoxy or $NO_2$; $R_3$ is halogen, methyl or methoxy; $R_5$ is halogen, $CF_3$, methoxy or $C_1$-$C_4$ alkyl; with the proviso that not more than two of said R groups may represent fluorine, and the salts thereof.

It has now been found that the compounds of formula:

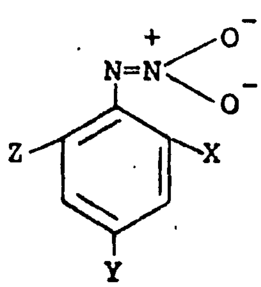 . $M^+$ (I)

wherein X, Y, Z and $M^+$ are defined above have the same or similar activity.

The present invention also relates to methods for treating graminaceous crops, leguminous crops, cotton, sunflowers and herbaceous ornamental plants, to increase axillary branching, improve the canopy and enhance flowering thereof. Surprisingly, application of the above-said salts to the foliage of the above-identified crops has the further advantage that said salts produce a dwarfing effect in said plants while increasing the stem stiffness thereof. They also enhance yield therefrom.

The present invention further relates to novel compounds of the following formula:

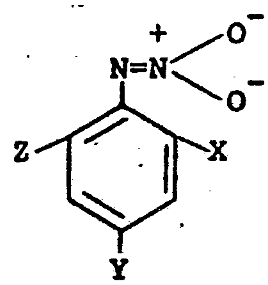

• $M^+$ (I)

wherein X, Y and Z are as defined above with the proviso that when X, Y and Z each are bromo, them $M^+$ cannot be Na, K, Ba or Ag.

For the preparation of the various formulations of the compounds of this invention please see the parent application.

The formula (I) salts of the symmetrical trisubstituted-N-nitroaniline compounds can be conventiently prepared from the trisubstituted nitroaniline compounds by one or more procedures, hereinafter referred to as Methods A,B,C,D or E.

<u>Methods for the preparation of salts of 2,4,6-trisubstituted N-nitroanilines</u>

<u>Method A</u>

The N-nitroaniline is dissolved in dilute aqueous sodium or potassium hydroxide and filtered. To this solution is added one molar equivalent of the appropriate amine salt and dissolved in water. In most cases the product separates

as a solid, and is removed by filtration and purified by recrystallization. Whenever no precipitation occurs or the product separates as an oil, an extraction with methylene chloride is performed. Concentration of this extract yields the product which may be further purified by recrystallization.

Method B

A solution of the N-nitroaniline in anhydrous ether is treated with a solution of one equivalent of the appropriate nitrogen base in ether. The desired product is removed by filtration and purified by washing and recrystallization. Precipitation of the product may be promoted by the addition of petroleum ether.

Method C

To a methanolic suspension of the silver salt of the N-nitroamine is added one equivalent of the halide salt of the appropriate amine dissolved in methanol. The mixture is stirred at 25°C for one hour. The precipitated silver halide is removed by filtration, the product recovered by evaporating the methanolic solution and purified by recrystallization.

Method D

An aqueous solution of the sodium salt of the N-nitroaniline is saturated with a large excess of the desired metal salt until solids remain undissolved. After stirring for one hour, the solids are removed by filtration and recrystallized or washed with a suitable solvent, and dried.

Method E

A methylene chloride solution of the free N-nitroaniline is treated with a three-fold excess of an aqueous solution of the appropriate metal hydroxide, acetate or carbonate. If precipitation occurs, the product is removed by filtration and recrystallized. Otherwise, the aqueous layer is separated and evaporated to dryness. The residue is treated with ethyl acetate and filtered. Removal of the ethyl acetate affords the product which may be recrystallized, if necessary.

. Surprisingly, it has also been found that the formula I compounds of this invention are effective for reducing the relative stem growth of broadleaf plants and for increasing the stem stiffness thereof, particularly for broadleaf agronomic crops such as sunflowers, when said compounds are applied preemergence to soil in which seeds of said plants have been sown. When used in this manner said formula I compounds are generally applied at rates of from 0.25 to 0.75 kg/ha.

The invention is further illustrated by the Examples set forth below.

EXAMPLE 1

Methods for the preparation of salts of 2,4,6-trisubstituted N-nitroanilines

Method A

The N-nitroaniline is dissolved in dilute aqueous sodium or potassium hydroxide and filtered. To this solution is added one molar equivalent of the appropriate amine salt and dissolved in water. In most cases the product separates as a solid, and is removed by filtration and purified by recrystallization. Whenever no precipitation occurs or the product separates as an oil, an extraction with methylene chloride is performed. Concentration of this extract yields the product which may be further purified by recrystallization.

Method B

A solution of the N-nitroaniline in anhydrous ether is treated with a solution of one equivalent of the appropriate nitrogen base in ether. The desired product is removed by filtration and purified by washing and recrystallization. Precipitation of the product may be promoted by the addition of petroleum ether.

Method C

To a methanolic suspension of the silver salt of the N-nitroamine is added one equivalent of the halide salt of the appropriate amine dissolved in methanol. The mixture is stirred at 25°C for one hour. The precipitated silver

halide is removed by filtration, the product recovered by evaporating the methanolic solution and purified by recrystallization.

Method D

An aqueous solution of the sodium salt of the N-nitroaniline is saturated with a large excess of the desired metal salt until solids remain undissolved. After stirring for one hour, the solids are removed by filtration and recrystallized or washed with a suitable solvent, and dried.

Method E

A methylene chloride solution of the free N-nitroaniline is treated with a three-fold excess of an aqueous solution of the appropriate metal hydroxide, acetate or carbonate. If precipitation occurs, the product is removed by filtration and recrystallized. Otherwise, the aqueous layer is separated and evaporated to dryness. The residue is treated with ethyl acetate and filtered. Removal of the ethyl acetate affords the product which may be recrystallized, if necessary.

By the above methods a number of compounds are prepared, these are listed in Tables appended hereto.

**TABLE I**

Inorganic salts of 2,4,6-tribromo-N-*aci*-nitroaniline

| No | $M^+$ | Analysis | | M.P. | Method |
|---|---|---|---|---|---|
| | | Calculated | Found | ($^oC$) | |
| 1 | $Na^+$ | C 16.64<br>H 1.40<br>N 6.47 | C 16.74<br>H 1.31<br>N 6.40 | 260 (dec) | E |
| 2 | $K^+$ | C 16.72<br>H 0.94<br>N 6.50 | C 17.19<br>H 0.74<br>N 6.26 | >230 | E |
| 3 | $Li^+$ | H 1.61<br>N 7.03<br>Br 60.12<br>Li 1.74 | H 1.46<br>N 6.90<br>Br 60.43<br>Li 1.59 | 284-288 (dec) | E |
| 4 | $Ca^{2+}$ | C 16.78<br>H 1.40<br>N 6.52<br>Br 55.82 | C 16.29<br>H 1.08<br>N 6.34<br>Br 56.03 | 240 (dec) | D |

Table I (continued)

| No | $M^+$ | Analysis | | M.P. ($^oC$) | Method |
|---|---|---|---|---|---|
| | | Calculated | Found | | |
| 5 | $Mg^{2+}$ | C 16.37<br>H 1.83<br>N 6.37<br>Mg 2.76 | C 16.62<br>H 2.45<br>N 5.71<br>Mg 3.27 | 129-135 (dec) | E |
| 6 | Ba | C 15.65<br>H 0.88<br>N 6.08<br>Ba 14.91 | C 15.37<br>H 0.86<br>N 6.07<br>Ba 14.96 | 202-208 | D |
| 7 | $Sr^{2+}$ | | | 216-219 (dec) | D |
| 8 | $Mn^{2+}$ | | | 142-146 (dec) | C |
| 9 | $Fe^{3+}$ | C 16.82 | C 16.34 | 136-138 (dec) | |

Table I (continued)

| No | $M^+$ | Analysis | | M.P. ($^oC$) | Method |
|---|---|---|---|---|---|
| | | Calculated | Found | | |
| 10 | $Co^{2+}$ | N 6.65<br>Co 6.99 | N 6.36<br>Co 6.79 | 172-<br>176<br>(dec) | C |
| 11 | Ni | | | | |
| 12 | $Cu^{2+}$ | H 0.50<br>N 6.91 | H 0.80<br>N 6.99 | 136-<br>139<br>(dec) | D |
| 13 | $Zn^{2+}$ | C 17.73 | C 18.16 | 110-<br>130<br>(dec) | D |
| 14 | $Al^{3+}$ | | | | * |
| 15 | Pb | | | | |
| 16 | $Ag^+$ | | | 176-<br>190<br>(dec) | D |

* = Prepared from aluminum isopropoxide and the N-nitroaniline in isopropyl alcohol

**TABLE I**

Inorganic salts of formula (I) compounds of structure

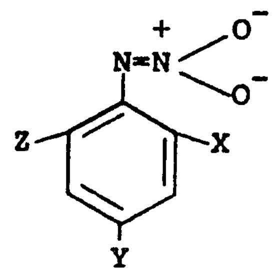

| No | X | Y | Z | $M^+$ | Analysis Calculated | Analysis Found | M.P. (°C) | Method |
|---|---|---|---|---|---|---|---|---|
| 1 | $CO_2C_2H_5$ | Br | Br | $K^+$ | C 25.72<br>H 2.13<br>N 6.60 | C 25.32<br>H 2.02<br>N 6.40 | 232-234 (dec) | E |
| 2 | Br | Br | I | $Na^+$ | | | | |
| 3 | Cl | I | Cl | $Na^+$ | | | | |

Table I (continued)

| No | X | Y | Z | $M^+$ | Analysis | | M.P. (°C) | Method |
|---|---|---|---|---|---|---|---|---|
| | | | | | Calculated | Found | | |
| 4 | $CF_3$ | Br | Br | $Na^+$ | C 20.81<br>H 1.00<br>N 6.94<br>Br 39.56<br>F 14.40<br>Na 5.70 | C 21.05<br><br>N 6.48<br>Br 39.47<br>F 14.13<br>Na 5.87 | 223-<br>226<br>(dec) | E |
| 5 | $CH_3$ | $CH_3$ | $CH_3$ | $Na^+$ | | | | |
| 6 | Cl | Cl | CL | $Na^+$ | | | | |
| 7 | Cl | Cl | Br | $Na^+$ | | | | |
| 8 | Br | CN | Br | $Na^+$ | | | 7250 | E |
| 9 | Br | $CH_3$ | Br | $Na^+$ | | | 7230 | E |

TABLE Ia

Amine salts with 2,4,6-tribromo-N-*aci*-nitroaniline

| No | $M^+$ | Analysis: Calculated | Analysis: Found | M.P. (°C) | Method |
|---|---|---|---|---|---|
| 1 | *n*-$C_{18}H_{37}NH_3^+$ | C 44.73<br>H 6.57<br>N 6.52<br>Br 37.21 | C 45.08<br>H 6.65<br>N 6.71<br>Br 37.57 | 75-77 | B |
| 2 | $H_2C{=}CH{-}CH_2NH_3^+$ | | | | |
| 3 | $HC{=}C{=}CH_2{-}NH_3^+$ | | | 136<br>138 | B |
| 4 | $(CH_3)_2CH_2CH_2CH(CO_2CH_3)NH_3^+$<br>L- | C 30.02<br>H 3.49<br>N 8.08<br>Br 46.10 | C 30.26<br>H 3.52<br>N 7.99<br>Br 46.21 | 129-<br>131 | B |
| 5 | $CH_3S(CH_2)_3NH_3^+$ | | | 102<br>103 | B |
| 6 | $C_8H_{17}CH{=}CH{-}C_8H_{16}{-}NHC_3H_6NH_3^+$ | N 6.77 | N 6.73 | 90-96 | B |
| 7 | 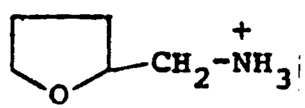 | | | 154<br>155 | |

TABLE Ib

Salts of Formula:

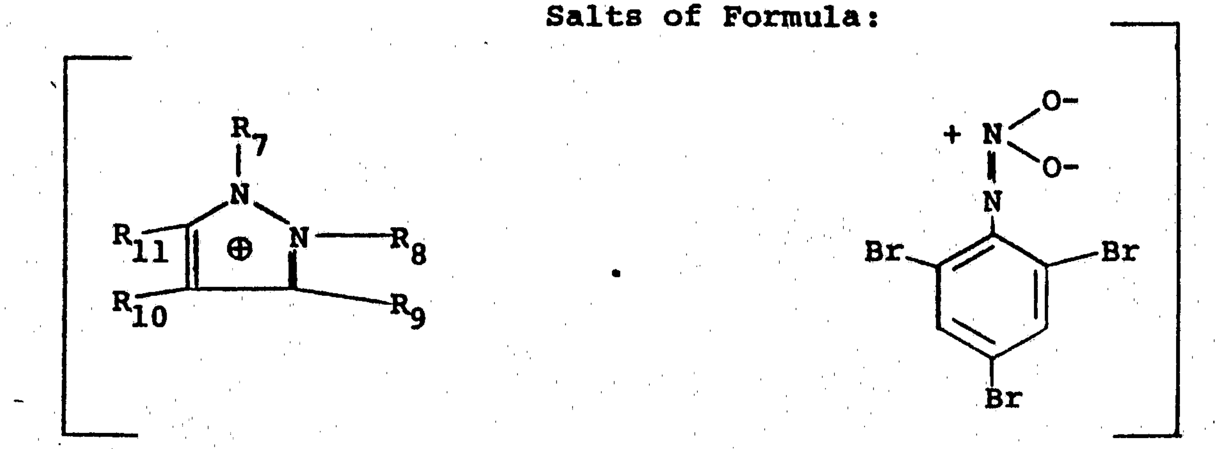

| No | $R_7$ | $R_8$ | $R_9$ | $R_{10}$ | $R_{11}$ | Analysis Calculated | Analysis Found | M.P (°C) |
|---|---|---|---|---|---|---|---|---|
| 1 | $CH_3$ | $CH_3$ | $C_6H_5$ | H | $C_6H_5$ | C 44.33<br>H 3.07<br>N 8.99<br>Br 38.47 | C 44.65<br>H 3.21<br>N 8.80<br>Br 38.44 | 145-146-(dec) |
| 2 | $CH_3$ | $CH_3$ | $C_6H_5$ | $OCH_3$ | $C_6H_5$ | C 44.13<br>H 3.24<br>N 8.58 | C 44.11<br>H 3.45<br>N 8.42 | 45-52 |

Table Ib (continued)

| No | $R_7$ | $R_8$ | $R_9$ | $R_{10}$ | $R_{11}$ | Analysis: Calculated | Analysis: Found | M.P (°C) |
|---|---|---|---|---|---|---|---|---|
| 3 | $CH_3$ | $CH_3$ | $4\text{-}F\text{-}C_6H_4\text{-}$ | H | $C_6H_5$ | C 41.91<br>H 3.06<br>N 8.50<br>Br 36.37<br>F 2.88 | C 42.02<br>H 3.12<br>N 8.52<br>Br 36.41<br>F 2.93 | 91-92 |
| 4 | $CH_3$ | $CH_3$ | 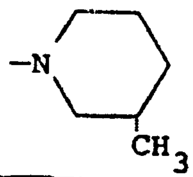 | H | $C_6H_5$ | C 42.88<br>H 4.07<br>N 10.87<br>Br 37.21 | C 43.12<br>H 4.11<br>N 10.91<br>Br 36.89 | 122-124 |
| 5 | $CH_3$ | $CH_3$ | 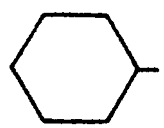 | H | $C_6H_5$ | C 43.90<br>H 4.01<br>N 8.09<br>Br 38.10 | C 43.94<br>H 4.14<br>N 8.84<br>Br 37.96 | 153-154 |
| 6 | $CH_3$ | $CH_3$ | $C_6H_5$ | $CH_3$ | $C_6H_5$ | C 45.24<br>H 3.32<br>N 8.79 | C 45.48<br>H 3.41<br>N 8.93 | 146-148 |

Table Ib (continued)

| No | $R_7$ | $R_8$ | $R_9$ | $R_{10}$ | $R_{11}$ | Analysis | | M.P ($^{o}C$) |
|---|---|---|---|---|---|---|---|---|
| | | | | | | Calculated | Found | |
| 7 | $C_2H_5$ | $CH_3$ | $C_6H_5$ | H | $C_6H_5$ | | | 139-140 (dec) |
| 8 | $CH_3$ | $CH_3$ | 4-Cl-$C_6H_4$- | H | $C_6H_5$ | | | |
| 9 | $CH_3$ | $CH_3$ | 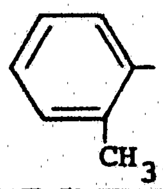 | H | $C_6H_5$ | | | |
| 10 | $CH_3$ | $CH_3$ | 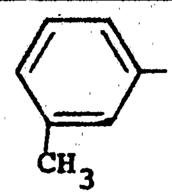 | H | $C_6H_5$ | | | |

Table Ib (continued)

| No | $R_7$ | $R_8$ | $R_9$ | $R_{10}$ | $R_{11}$ | Analysis | | M.P ($^oC$) |
|---|---|---|---|---|---|---|---|---|
| | | | | | | Calculated | Found | |
| 11 | $CH_3$ | $CH_3$ | 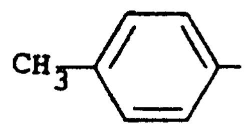 | H | $C_6H_5$ | | | |
| 12 | $CH_3$ | $CH_3$ | 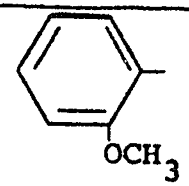 | H | $C_6H_5$ | | | |
| 13 | $CH_3$ | $CH_3$ | 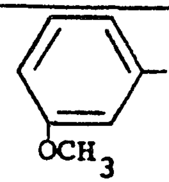 | H | $C_6H_5$ | | | |
| 14 | $CH_3$ | $CH_3$ | 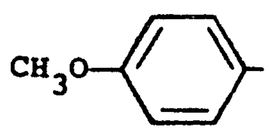 | H | $C_6H_5$ | | | |

**Table Ib (continued)**

| No | $R_7$ | $R_8$ | $R_9$ | $R_{10}$ | $R_{11}$ | Analysis | | M.P ($^oC$) |
|---|---|---|---|---|---|---|---|---|
| | | | | | | Calculated | Found | |
| 15 | $CH_3$ | $CH_3$ | $C_6H_5$ | F | $C_6H_5$ | | | |
| 16 | $CH_3$ | $CH_3$ | $C_6H_5$ | Cl | $C_6H_5$ | | | |
| 17 | $CH_3$ | $CH_3$ | $C_6H_5$ | Br | $C_6H_5$ | | | |
| 18 | $CH_3$ | $CH_3$ | 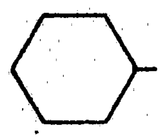 | H | 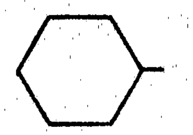 | | | |
| 19 | $CH_3$ | $CH_3$ | 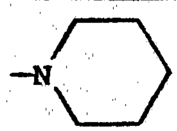 | H | $C_6H_5$ | | | |
| 20 | $CH_3$ | $CH_3$ | H | H | H | | | |

## Table Ic

Salts of Formula:

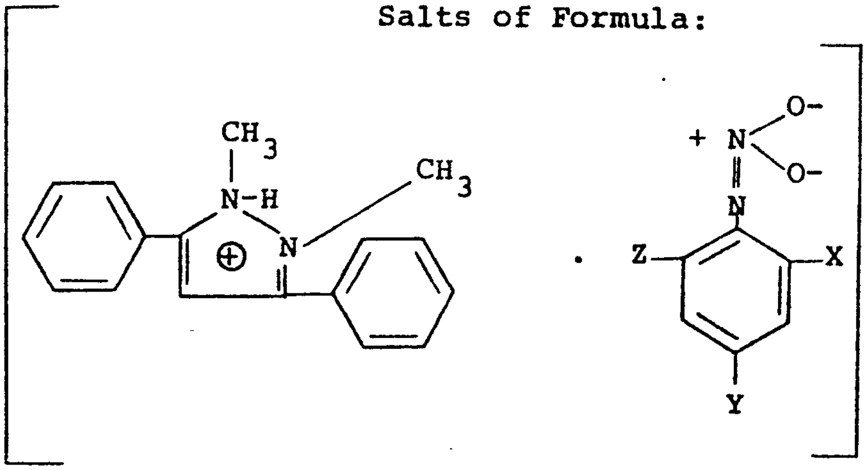

| X | Y | Z | M.P. |
|---|---|---|---|
| Br | Br | Br | 89-92 |

## Table Id

Salts of Formula:

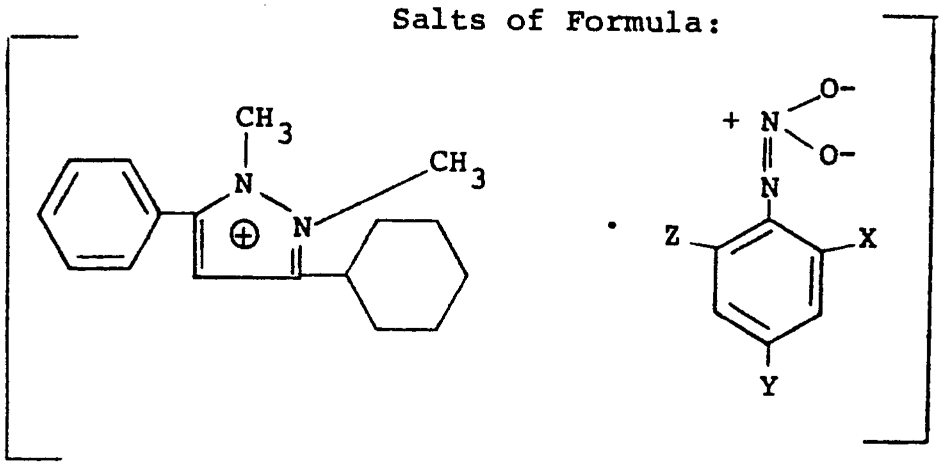

| X | Y | Z | M.P. |
|---|---|---|---|
| Br | Br | Br | |

Table Ie

Salts of Formula:

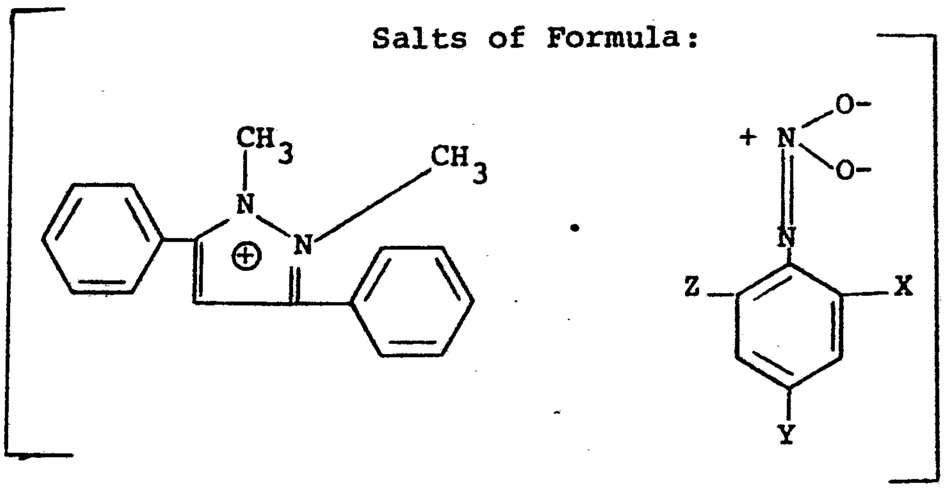

| No | X | Y | Z | Analysis Calculated | Analysis Found | M.P (°C) |
|---|---|---|---|---|---|---|
| | | | | C 45.86 | C 46.16 | |
| 1 | $CF_3$ | Br | Br | H 3.36 | H 3.19 | 126- |
| | | | | N 8.89 | N 9.01 | 127 |
| | | | | F 9.04 | F 9.23 | (dec) |
| | | | | C 47.08 | C 47.24 | |
| 2 | Br | $CF_3$ | Br | H 3.13 | H 3.20 | 140- |
| | | | | N 9.15 | N 9.08 | 142 |
| | | | | F 9.31 | F 9.46 | (dec) |
| | | | | Br 26.11 | Br 26.11 | |
| | | | | C 55.39 | C 55.69 | |
| 3 | Cl | Cl | Cl | H 4.04 | H 3.92 | 105- |
| | | | | N 11.23 | N 11.02 | 106 |
| | | | | C 41.22 | C 41.51 | |
| 4 | I | Br | Br | H 2.86 | H 3.00 | 142- |
| | | | | N 8.36 | N 8.35 | 143 |
| | | | | Br 23.85 | Br 23.56 | (dec) |
| | | | | C 69.55 | C 69.19 | |
| 5 | $CH_3$ | $CH_3$ | $CH_3$ | H 6.86 | H 6.83 | 88-91 |
| | | | | N 12.30 | N 12.06 | 91 |
| | | | | C 47.73 | C 47.51 | |
| 6 | Br | Cl | Br | H 3.31 | H 3.35 | 128- |
| | | | | N 9.68 | N 9.66 | 132 |

EXAMPLE 2

Evaluation of 2,4,6-trisubstituted-N-nitroaniline salts as Dwarfing and Stem Stiffening agents for Barley

In the following tests, test coumpounds are dissolved or dispersed in acetone-water (1:1) mixtures at the final concentration corresponding to the kg/ha rates indicated in the table below. The solution also contains 0.25% v/v colloidal BIOFILM® (a product of Colloidal Products Corp.) which is a mixture of alkyl aryl polyethoxyethanol, free and combined fatty acids, glycol ethers, dialkylbenzene carboxylate and 2-Propanol.

The plant species used in these tests are barley (*Hordeum vulgare*), (*Mexico*) and soybean (*Glycine max*) Adelphia.

The solution or dispersion of the compound under test is sprayed at a rate of 747 l/ha with a moving nozzle along an overhead stationary track. The spray nozzle moves at a constant speed over the plants.

The plants are grown in plastic pots, and are well established at the time of treatment. The seedlings of barley are 10 to 20 cm in height, while the seedlings of soybeans are at the second to third trifoliate stage. Plants are watered prior to treatment and then sprayed to provide the kg/ha rate of test compound desired. After spraying the plants are placed on greenhouse benches and watered and fertilized in accordance with normal greenhouse procedures.

Three weeks after spraying the plants are measured and harvested. Harvesting is done at the boot stage. All treatments are replicated six times and comparisons are made against untreated controls. Data obtained are reported in Tables II and III below as percent reduction in plant height over untreated controls.

TABLE II

Evaluation of test compounds as
Barley dwarfing agents

| Compound | Rate kg/ha | % Reduction in Plant Height over untreated controls |
|---|---|---|
| 1,2-Dimethyl-3,5-diphenyl-pyrazolium salt with 2,4,6-Tribromo-N-*aci*-nitroaniline | 1.5 | 15 |
| | 0.50 | 8.7 |
| | 0.17 | 37.8 |
| 4-Methoxy-1,2-dimethyl-3,5-diphenylpyrazolium salt with 2,4,6-tribromo-N-*aci*-nitroaniline | 2.24 | >60 |
| | 0.56 | >20<35 |
| | 0.11 | >10<15 |
| 2,4,6-Tribromo-N-nitroaniline Potassium salt | 1.5 | 2.0 |
| | 0.50 | 11.8 |
| | 0.17 | 2.7 |
| 2,4,6-Tribromo-N-nitroaniline sodium salt | 1.5 | 13.0 |
| | 0.50 | 13.0 |
| | 0.17 | 6.2 |
| 2,4,6-Tribromo-N-nitro-aniline magnesium salt | 2.0 | >10<15 |
| | 1.0 | >10<15 |
| | 0.5 | >10<15 |
| 2,4,6-Tribromo-N-nitro-aniline Calcium salt | 2.0 | >10<15 |
| | 1.0 | >10<15 |
| | 0.5 | >10<15 |

TABLE 3

Evaluation of test compounds as Dwarfing agents and stem stiffening agents for soybeans

| Compound | Rate Kg/ha | % Reduction in Plant Height over untreated controls |
| --- | --- | --- |
| 2,4,6-Tribromo-N-nitro-aniline sodium salt | 0.25<br>0.125 | >40<br>>35 |
| 2,4,6-Tribromo-N-nitro-aniline potassium salt | 0.50<br>0.25<br>0.125 | >30<br>>5<br>>5 |
| 2,4,6-Tribromo-N-nitro-aniline magnesium salt | 2.0<br>1.0<br>0.5 | >10<15<br>>10<15<br>>20<35 |
| 2,4,6-Tribromo-N-nitro-aniline calcium salt | 2.0<br>1.0<br>0.5 | >10<15<br>>10<15<br>>10<15 |
| 1-Hexadecylpyridinium salt with 2,4,6-Tribromo -N-*aci*-nitroaniline | 0.5<br>0.25<br>0.125 | >50<br>>35<br>>35 |
| 1,1-Dimethylpiperidinium salt with 2,4,6-Tribromo-N-*aci*-nitroaniline | 0.5<br>0.25<br>0.125 | >50<br>>35<br>>25 |
| 1,2-Dimethyl-3,5-diphenyl-pyrazolium salt with 2,4,6-tribromo-N-*aci*-nitroaniline | 0.5<br>0.25<br>0.125 | >20<35<br>>10<15<br>normal |
| 4-Methoxy-1,2-dimethyl-3,5-diphenylpyrazolium salt with 2,4,6-tribromo-N-*aci*-nitroaniline | 0.5<br>0.25<br>0.125 | >35<br>>35<br>>10 |
| 3-(*p*-Fluorophenyl)-1,2-dimethyl-5-phenylpyrazolium salt with 2,4,6-tribromo-N-*aci*-nitroaniline | 0.5<br>0.25<br>0.125 | >25<br>>25<br>>20 |
| 1,2-Dimethyl-3-(methylpiperidino)-5-phenylpyrazolium salt with 2,4,6-tribromo-N-*aci*-nitroaniline | 0.5<br>0.25<br>0.125 | >25<br>>25<br>>20 |

Table 3 (continued)

| Compound | Rate kg/ha | % Reduction in Plant Height over untreated controls |
|---|---|---|
| 1,2-Dimethyl-3,5-diphenyl-pyrazolium salt with 2,4,6-trichloro-N-*aci*-nitro-aniline | 0.5<br>0.25<br>0.125 | >25<br>>15<br>>10 |
| 1,2-Dimethyl-3,5-diphenyl-pyrazolium salt with 2,4-dibromo-6-iodo-N-*aci*-nitroaniline | 0.5<br>0.25<br>0.125 | >25<br>>15<br>>10 |
| *cis*-1,2-Dimethyl-3,5-diphenyl-pyrazolidinium salt with 2,4,6-tribromo-N-nitroaniline | 0.5<br>0.25<br>0.125 | >60<br>>30<br>>20 |
| 1,2-Dimethyl-3,5-diphenyl-pyrazolium salt with 2,4,6-Trimethyl-N-*aci*-nitroaniline | 0.5<br>0.25<br>0.125 | >10<br>>5<br>slight increase |
| 3-Cyclohexyl-1,2-dimethyl-5-phenylpyrazolium salt with 2,4,6-tribromo-N-*aci*-nitro-aniline | 0.5<br>0.25<br>0.125 | >40<br>>20<br>>10 |
| 1,2-Dimethyl-3,5-diphenyl-pyrazolium salt with 2,6-dibromo-4-chloro-N-*aci*-nitroaniline | 0.5<br>0.25<br>0.125 | >40<br>>30<br>>25 |
| 1,2-Dimethyl-3,5-diphenyl-pyrazolium salt with 4,6-dibromo-α,α,α-trifluoro-N-*aci*-nitro-*o*-toluidine | 0.5<br>0.25<br>0.125 | >40<br>>40<br>>20 |
| 1,2-Dimethyl-3,5-diphenyl-pyrazolium salt with 2,6-dibromo-α,α,α-trifluoro-N-*aci*-nitro-*o*-toluidine | 0.5<br>0.25<br>0.125 | >20<br>>15<br>>10 |

## EXAMPLE 3

**Evaluation of test compounds for Increasing axillary Branching and improving the canopy of Broad leaf plants using Soybeans (*Glycine max*) Adelphia variety as the plant species**

In these tests seedling soybean plants approximately three weeks old, and grown in plastic pots to the 2nd to 3rd trifoliate stage, are sprayed with solutions or dispersions of test compound prepared as described in Example 2 above. The same spraying apparatus, described in Example 2 above, is used and after spraying the plants are placed on greenhouse benches and watered and fertilized. Three weeks after treatment the plants are examined to determine what effect the application of test compound has had on the axillary branching and canopy development of the treated plants.

Increased axillary branching and improved canopy are biological responses which are highly advantageous in the growing of ornamental plants. These responses also have advantage in many crops since increased branching may result in the greater production of fruit, while improved canopy tends to shade and suppress the growth of undesirable weeds that compete with the crops for sunlight, water and plant nutrients. Data obtained are reported in Table IV below where axillary branching and canopy are reported as percent increase over untreated controls.

TABLE IV

Evaluation of test compounds for increasing axillary branching and improving the canopy of treated broad leaf plants using soybeans (var. Adelphia) as the plant species

| Compound | Rate kg/ha | % Increase in axillary branching over untreated controls | % Increase in Plant Canopy over untreated controls |
|---|---|---|---|
| 1,2-Dimethyl-3,5-diphenyl-pyrazolium salt with 2,4,6-tribromo-N-aci nitroaniline | 0.5 | >10<15 | >10<15 |
| | 0.25 | >10<15 | >20<35 |
| | 0.125 | >10<15 | >10<15 |
| 4-Methoxy-1,2-dimethyl-3,5-diphenylpyrazolium salt with 2,4,6-Tribromo-N-aci-nitroaniline | 0.5 | >10<15 | >20<35 |
| | 0.25 | normal | >20<35 |
| | 0.125 | normal | normal |
| 3-(p-Fluorophenyl)-12,-dimethyl -5-phenylpyrazolium salt with 2,4,6-tribromo-N-aci-nitroaniline | 0.5 | normal | >10<15 |
| | 0.25 | >10<15 | >10<15 |
| | 0.125 | normal | normal |
| 1,2-Dimethyl-3,5-diphenylpyrazolium salt with 4,6-dibromo-α,α,α-trifluoro-N-aci-O-toluidine | 0.5 | >10<15 | >10<15 |
| | 0.25 | >10<15 | >10<15 |
| | 0.125 | >10<15 | >20<35 |

TABLE IV (continued)

| Compound | Rate kg/ha | % Increase in axillary branching over untreated controls | % Increase in Plant Canopy over untreated controls |
|---|---|---|---|
| 1,2-Dimethyl-3,5-diphenylpyrazolium salt with 2,4-dibromo-6-iodo-N-aci-nitroaniline | 0.5 | >20<35 | >20<35 |
| | 0.25 | >20<35 | >20<35 |
| | 0.125 | >10<15 | >10<15 |
| 1,2-Dimethyl-3,5-diphenylpyrazolium salt with 2,4,6-trimethyl-N-aci-nitroaniline | 0.5 | >10<15 | >10<15 |
| | 0.25 | >10<15 | >10<15 |
| | 0.125 | normal | normal |
| 3-Cyclohexyl-1,2-dimethyl-5-phenylpyrazolium salt with 2,4,6-tribromo-N-aci-nitroaniline | 0.5 | >10<15 | slight reduction |
| | 0.25 | >10<15 | >10<15 |
| | 0.125 | >10<15 | >10<15 |
| 1,2-Dimethyl-3,5-diphenyl-pyrazolium salt with 2,6-dibromo-4-chloro-N-aci-nitroaniline | 0.5 | >10<15 | >10<15 |
| | 0.25 | >10<15 | >10<15 |
| | 0.125 | >10<15 | normal |

TABLE IV (continued)

| Compound | Rate kg/ha | % Increase in axillary branching over untreated controls | % Increase in Plant Canopy over untreated controls |
|---|---|---|---|
| 2,4,6-Tribromo-N-nitroaniline sodium salt | 0.50 | >20<35 | >10<15 |
| | 0.25 | >20<35 | >20<35 |
| | 0.125 | >20<35 | >20<35 |
| 2,4,6-Tribromo-N-nitroaniline potassium salt | 0.50 | >20<35 | normal |
| | 0.25 | normal | >10<15 |
| | 0.125 | normal | >10<15 |
| 2,4,6-Tribromo-N-*aci*-nitro-aniline Calcium salt (2:1) | 0.5 | >20<35 | moderate reduction |
| | 0.25 | >20<35 | slight reduction |
| | 0.125 | >20<35 | >10<15 |
| 2,4,6-Tribromo-N-*aci*-nitro-aniline magnesium salt (2:1) | 0.5 | >20<35 | moderate reduction |
| | 0.25 | >20<35 | moderate reduction |
| | 0.125 | >10<15 | >10<15 |
| 2,4,6-Tribromo-N-nitro aniline with *cis* 1,2-dimethyl-3,5-diphenylpyrazolidine | 0.5 | >20<35 | moderate reduction |
| | 0.25 | >20<35 | moderate reduction |
| | 0.125 | >10<15 | >20<35 |

TABLE IV (continued)

| Compound | Rate kg/ha | % Increase in axillary branching over untreaed controls | % Increase in Plant Canopy over untreated controls |
|---|---|---|---|
| Tributyl(2,4-dichlorobenzyl) phosphonium salt with 2,4,6-Tribromo-N-*aci*-nitroaniline | 0.5 | >20<35 | moderate reduction |
| | 0.25 | >10<15 | >10<15 |
| | 0.125 | normal | normal |
| 1-Hexadecylpyridinium salt with 2,4,6-tribromo-N-*aci*-nitroaniline | 0.5 | normal | >10<15 |
| | 0.25 | normal | >10<15 |
| | 0.125 | normal | normal |
| Methyltriphenylphosphonium salt with 2,4,6-tribromo-N-*aci*-nitroaniline | 0.5 | >10<15 | >20<35 |
| | 0.25 | normal | >10<15 |
| | 0.125 | normal | normal |

EXAMPLE 4

Evaluation of test compounds to determine their effectiveness for increasing flowering of plants using soybeans (*Glycine max*) var. Adelphia as the plant species

In the following tests seedling soybean plants growing in plastic cups are sprayed with aqueous-acetone solutions or dispersions of test compounds when they have reached the 2nd to 3rd trifoliate stage. Test solutions or dispersions are prepared with 0.25 v/v Colloidal BIOFILM® surfactant and sufficient test compound to provide the plants with 0.5, 0.25, 0.125 or 0.06 kg/ha of said compound when the test solutions are sprayed on said plants from an overhead sprayer delivering 747 l/ha of solution.

After spraying the plants are placed on greenhouse benches and watered and fertilized. Three weeks after spraying the plants are examined and the amount of flowering determined for all plants. Data obtained are reported in the table below as percent increase in flowering over untreated controls.

From the data reported in Table V below it can be seen that the compounds of the present invention applied at rates of from 0.06 to 0.25 kg/ha enhance flowering of treated plant more than 10% over untreated controls. This response is particularly desirable for treatment of flowering varieties of ornamental plants as well as agronomic crops.

TABLE V

Evaluation of test compounds to determine their effectiveness for increasing flowering of the treated plants

| Compound | Rate kg/ha | % Increase in Flowering over untreated controls |
|---|---|---|
| 1,2-Dimethyl-3,5-diphenyl-pyrazolium salt with 2,4,6-tribromo-N-aci-nitroaniline | 0.25 | >10<15 |
| | 0.125 | >20<35 |
| | 0.06 | >10<15 |
| 4-Methoxy-1,2-dimethyl-3,5-diphenylpyrazolium salt with 2,4,6-tribromo-N-aci-nitroaniline | 0.5 | Moderate Reduction |
| | 0.25 | >10<15 |
| | 0.125 | >10<15 |
| 1,2-Dimethyl-3-(3-methylpiperidono)-5-phenyl salt with 2,4,6-tribromo-N-aci-nitroaniline | 0.5 | Slight Reduction |
| | 0.25 | >10<15 |
| | 0.125 | >10<15 |
| 1,2-Dimethyl-3,5-diphenylpyrazolium salt with 2,4-dibromo-6-iodo-N-aci-nitroaniline | 0.5 | normal |
| | 0.25 | >10<15 |
| | 0.125 | >10<15 |
| 1,1-Dimethylpiperidinium salt with 2,4,6-Tribromo-N-aci-nitroaniline | 0.5 | >20<35 |
| | 0.25 | >10<15 |
| | 0.125 | normal |
| Methyltriphenylphosphonium salt with 2,4,6-Tribromo-N-aci-nitro-aniline | 0.5 | >10<15 |
| | 0.25 | >10<15 |
| | 0.125 | normal |
| 2,4,6-Tribromo-N-nitroaniline sodium salt | 0.5 | normal |
| | 0.25 | >10<15 |
| | 0.125 | >10<15 |
| 2,4,6-Tribromo-N-nitroaniline potassium salt | 0.5 | normal |
| | 0.25 | >10<15 |
| | 0.125 | >10<15 |

EXAMPLE 5

Evaluation of test compounds for enhancing crop yield on cotton

In this experiment cotton plants (Gossypium hirsutum var. "Stoneville") are grown in 20 X 15 cm fibre pots to the 6th to 7th true leaf stage. They are then sprayed with test solutions or dispersions prepared in water-acetone mixtures containing (1) 0.25% v/v of colloidal BIOFILM® surfactant which is a mixture of alkyl aryl polyethoxyethanol, free and combined fatty acids, glycol ethers, dialkylbenzene carboxylate and 2-propanol, and (2) sufficient test compound to provide 1.5, 0.75 or 0.25 kg/ha thereof when applied to said plants from an overhead sprayer designed to deliver 747 l/ha with a moving nozzle over a stationary track. The spray nozzle moves at a constant speed over the test species.

After spraying the plants are placed on greenhouse benches and watered and fertilized in accordance with conventional greenhouse practices. Periodically, the plants are examined, measured, and the number of flower, buds and bolls determined for each plant. Data obtained are reported in Table VI below as percent increase in flowers, buds and bolls over untreated controls.

TABLE VI

Evaluation of 1,2-Dimethyl-3,5-diphenylpyrazolium salt with 2,4,6-Tribromo-N-*aci*-nitroaniline for Cotton Yield Enhancement (TREATED AT 6 TO 7TH TRUE LEAF STAGE)

| Days After Treatment | Rate (Kg/Ha) | % Height Reduction | % Increase of Visible Flowers and Buds |
|---|---|---|---|
| 33 | 0.25 | - | 29.3 |
| | 0.75 | 10.1 | 42.8 |
| | 1.50 | 21.8 | - |
| 42 | | | |
| | 0.25 | - | 25.9 |
| | 0.75 | 9.3 | 9.3 |
| | 1.50 | 7.5 | - |
| 54 | | | |
| | 0.25 | - | 9.2 |
| | 0.75 | 9.2 | 33.2 |
| | 1.50 | 15.0 | 9.6 |

| Days After Treatment | Rate (Kg/Ha) | % Increase | | |
|---|---|---|---|---|
| | | Flower Buds | Open Flowers | Bolls |
| 54 | 0.25 | 25.6 | 21.3 | 62.5 |
| | 0.75 | 25.6 | 32.8 | 41.3 |
| | 1.50 | - | 29.5 | - |

## EXAMPLE 6

### Evaluation of test compounds for enhancing crop yield in sunflowers

In this experiment the procedure of Example 5 was repeated excepting that the crop used was sunflowers, *Helianthus annuus* D0164 variety, the plants were sprayed three weeks after planting and at the two leaf stage treatments were made at 0.25, 0.125 and 0.0625 kg/ha of test compound.

The plants were held for 63 days after treatment when the number and weight of flowers was determined and recorded. Data obtained are reported in Table VII below.

TABLE VII

Evaluation of 1,2-Dimethyl-3,5-Diphenylpyrazolium salt with 2,4,6-Tribromo-N-*aci*-nitroaniline for sunflower uield enhancement (TREATED AT 2 TRUE LEAF STAGE)

| Days After Treatment | Rate (Kg/Ha) | % Height Reduction | Average Flower Number | % Increase of Flower Weights |
|---|---|---|---|---|
| 63 | | | | |
| | 0.0625 | - | 2.1 | 45.5 |
| | 0.125 | - | 3.8 | 3.7 |
| | 0.250 | 54.8 | 9.5 | 24.0 |

These data are especially significant since they demonstrate the uniqueness of the pyrazolium salts of the trisubstituted-N-nitroaniline compounds in producing multiple flowering of sunflowers.

## EXAMPLE 7

### Evaluation of test compounds for enhancing crop yield on soybeans

The procedure of Example 5 was repeated excepting that soybeans (var. Adelphia) were used as the crop plant and plants were treated at the 2nd to 3rd trifoliate stage and held on greenhouse benches for three weeks after treatment. Three weeks after treatment all plants were examined and the number of developed pods counted. Data obtained are

reported in Table VIII below.

TABLE VIII

Evaluation of 1,2-Dimethyl-3,5-diphenylpyrazolium salt with 2,4,6-tribromo-N-*aci*-nitroaniline for soybean yield enhancement

(TREATED AT 2ND TO 3RD TRIFOLIATE)

| Days After Treatment | Rate (kg/ha) | % Increase in Pods over untreated controls |
|---|---|---|
| 21 | 0.50 | >10 |
| | 0.25 | >10 |
| | 0.125 | >10 |
| | 0.063 | >10 |

28,162

CLAIMS:

1. A method for the control of relative growth of graminaceous crops, leguminous crops and cotton comprising applying to the foliage, stems,roots, seeds of the plants or to soil in which the crops are grown a compound having the formula:

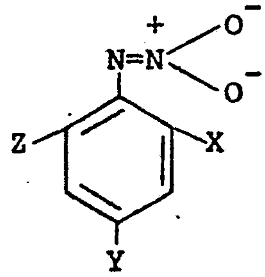

· $M^+$ (I)

wherein X, Y and Z are each halogen, $C_1$-$C_3$ alkyl, $C_1$-$C_3$ alkoxy, $-CO_2$alkyl ($C_1$-$C_3$), CN, $CF_3$, $-SO_2F$ or $-SO_2F_2$; $M^+$ is

(1) alkali metals, alkaline earth metals, Mn, Fe, CO, Ni, Cu, Zn, Al, Pb or Ag;

(2) the formula: $NR_1R_2R_3R_4$ (Ia)

wherein $R_1$ is hydrogen, $C_{13}$-$C_{18}$ alkyl straignt chain or branched or $C_3$-$C_6$ alkenyl, $C_3$-$C_6$ cycloalkyl, $R_2$ is hydrogen, $C_{13}$-$C_{18}$ alkyl straight chain or branched or $R_3$ is hydrogen, $C_{13}$-$C_{18}$ alkyl straight chain or branched $R_4$ is hydrogen, $C_{13}$-$C_{18}$ alkyl straight chain or branched or

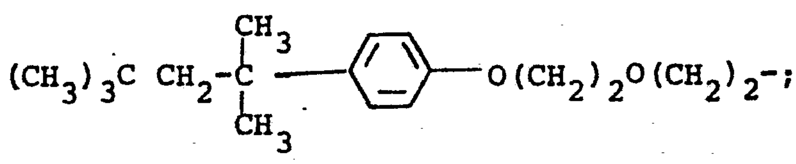

(3) the formula:

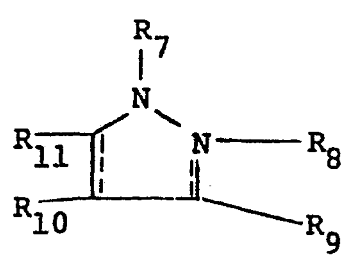

(Ib)

wherein $R_7$ and $R_8$ each are $C_1$-$C_3$alkyl; $R_{10}$ is hydrogen, halogen, $C_1$-$C_3$ alkyl or $C_1$-$C_3$ alkoxy; $R_9$ and $R_{11}$ each are hydrogen $C_1$-$C_6$ alkyl straight chain or branched, $C_3$-$C_6$ cycloalkyl,

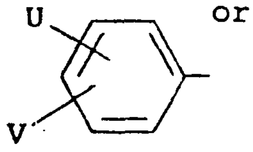 or 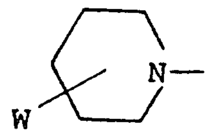 ; wherein U and V each are hydrogen, halogen, $C_1$-$C_3$ alkyl, $C_1$-$C_3$ alkoxy, CN or $CF_3$; W is hydrogen or $C_1$-$C_3$ alkyl; and each --- symbol represents a single or double bond, wherein if both are single bonds then the cations are pyrazolidinium cations represented by formula (Ic)

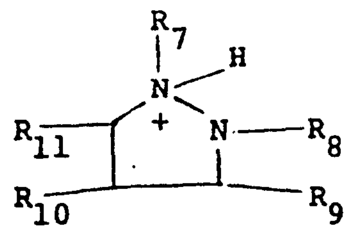

(Ic)

wherein $R_7$ to $R_{11}$ are as defined above; and when one of the --- symbols represents a double bond then the cations are pyrazolinium cations represented by formula (Id)

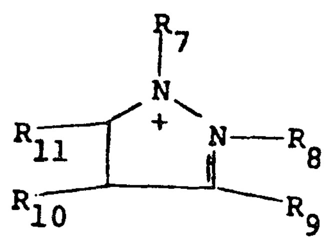

(Id)

wherein $R_7$ to $R_{11}$ are as defined above; and when both --- symbols represent double bonds, then the cations are pyrazolium cations represented by the formula (Ie)

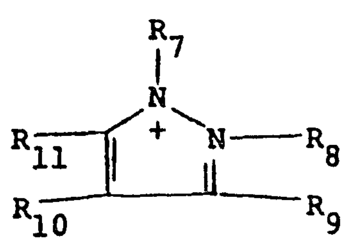

(Ie)

wherein $R_7$ to $R_{11}$ are as defined above;

(4) the formula:

$$R_{12}-P(R_{13})_3 \quad \text{(If)}$$

wherein $R_{12}$ is $C_1$-$C_6$ alkyl, 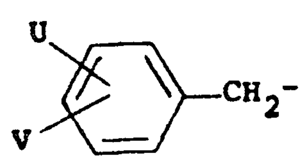 or 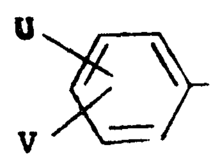

wherein U and V are as hereinabove defined; $R_{13}$ is $C_1$-$C_6$ alkyl or 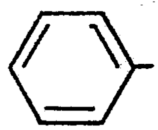 or

(5) the formula:

$$(C_6H_5)_3S \quad (Ig)$$

2. A method according to Claim 1, wherein the compound is 2,4,6-tribromo-N-nitroaniline sodium salt and the crop is barley.

3. A compound of the following formula:

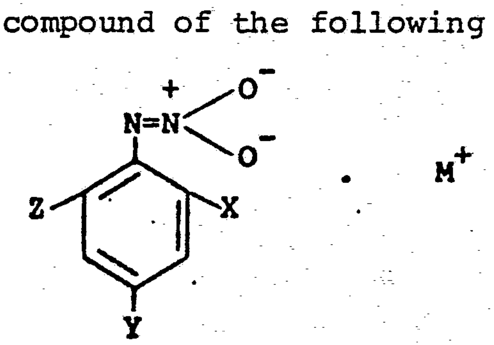

wherein X, Y and Z are as defined above with the proviso that when X, Y and Z each are bromo, then $M^+$ cannot be Na, K, Ba or Ag.

4. A process for the preparation of a compound of the following formula:

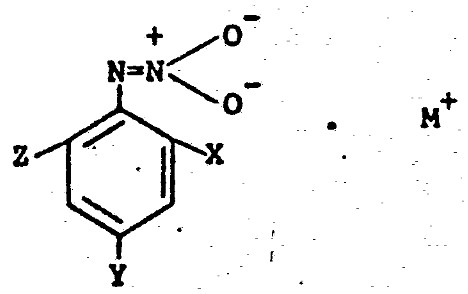

wherein X, Y and Z are as defined above with the proviso that when X, Y and Z each are bromo, then $M^+$ cannot be Na, K, Ba or Ag, comprising treating an alkaline solution of the free base

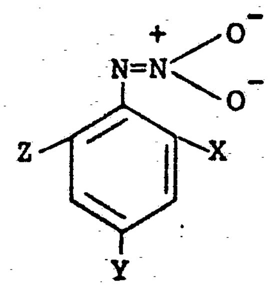

wherein X, Y and Z are as defined above with an equivalent amount of a salt of $M^+$ in the presence of an inert solvent selected of water, lower alcohols or ether.

5. A composition for inhibiting lodging of cotton, leguminous crops and graminaceous crops characterized by applying to the foliage of the crops or to soil containing seeds of the crops from about 0.01 to 1.5 kg/hectare of a compound having the formula:

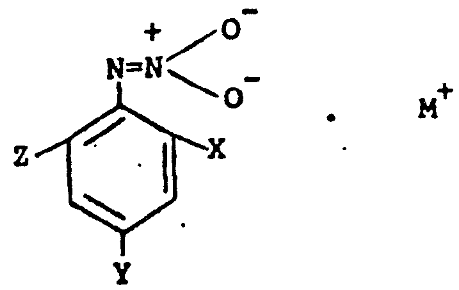

wherein X, Y and Z and $M^+$ are as defined above and a diluent of finely divided inert solid carriers, water, inert organic solvents or mixtures thereof.

6. A composition according to Claim 5 wherein the compound is 2,4,6-tribromo-N-nitroaniline sodium salt and the crop is barley.

7. A method for enhancing barley crops yield comprising applying to the foliage of the crop a yield enhancing amount of a compound having the formula:

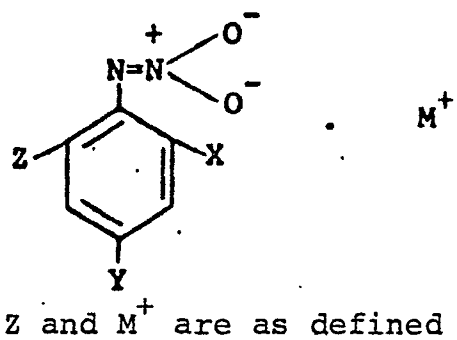

wherein X, Y and Z and $M^+$ are as defined above.

8. A method of increasing the crop yield in barley comprising applying to the foliage of the crop a yield enhancing amount of the compound 2,4,6-tribromo-N-nitroaniline.

9. A method for reducing the relative stem growth of broadleaf agronomic crops and increasing the stem stiffness thereof comprising applying preemergence to soil in which seeds of the crop are sown from 0.125 kg/ha to 2.0 kg/

ha of a compound of the formula:

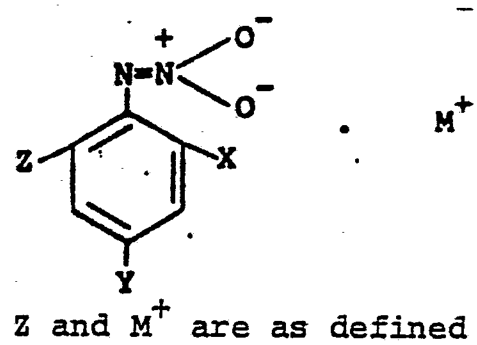

wherein X, Y and Z and $M^+$ are as defined above.

10. A method for inhibiting lodging in barley which comprises applying to the foliage of the plants a lodging inhibiting amount of a compound of the formula:

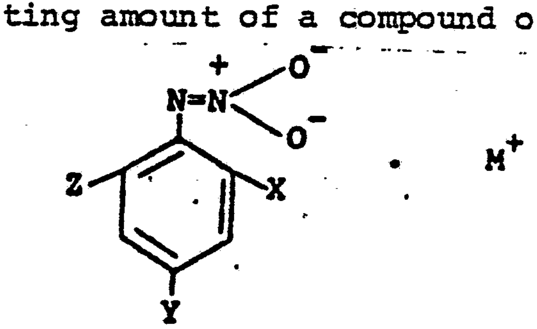

wherein X, Y and Z and $M^+$ are as defined above.